# EUROPÄISCHE PATENTANMELDUNG

(11) **EP 1 103 266 A2**
(43) Veröffentlichungstag der Anmeldung: **30.05.2001**
(21) Anmeldenummer: 00124135.5
(22) Anmeldetag: 07.11.2000
(51) Int. Cl.: A61K 35/78, A61K 9/14, A61K 9/51

(54) **Curcumin-Formulierungen**

(30) Priorität: 26.11.1999 DE 19956848
(71) Anmelder: BASF AKTIENGESELLSCHAFT, 67056 Ludwigshafen (DE)
(72) Erfinder: Auweter, Helmut, Dr., 67117 Limburgerhof (DE); Bohn, Heribert, 67319 Wattenheim (DE); Hasselwander, Oliver, Dr., 76829 Landau (DE); Rieger, Jens, Dr., 67069 Ludwigshafen (DE); Schröder, Hartwig, Dr., 69226 Nussloch (DE); Wegner, Christoph, Dr., 67281 Kirchheim (DE)

(57) **Zusammenfassung**

Die Erfindung betrifft Curcumin-Formulierungen, enthaltend Curcumin, dessen intensivste Reflexionslinie im Röntgen-Diffraktogramm im Bereich zwischen 2 Θ = 13° und 2 Θ = 15° liegt, deren Herstellung und Verwendung.

## Beschreibung

Die Erfindung betrifft Curcumin-Formulierungen, enthaltend Curcumin einer neuen Kristallmodifikation sowie deren Herstellung und Verwendung.

Curcumin, ein in der Natur, insbesondere in Gelbwurzgewächsen vorkommender Farbstoff, wird im Lebensmittelbereich häufig als Ersatz für synthetische Farbstoffe u.a. zum Färben von Gebäck, Pickles, Käse, Suppen, Salatdressings und Gewürzen verwendet.

Neben seiner Eigenschaft als Färbemittel besitzt Curcumin außerdem interessante pharmakologische Wirkungen, die sowohl im Tierversuch als auch am Menschen nachgewiesen wurden. Dazu gehören beispielsweise: cholikinetische Wirkung (Stimulierung des Gallenblasenmuskels); antihyperlipidämische Wirkung (Senkung des Plasma-Cholesterol-Spiegels); Antitumor-Wirkung [siehe dazu R. Hänsel, K. Keller, H. Rimpler und G. Schneider (Hrsg.) Hagers Handbuch der Pharmazeutischen Praxis, Springer Verlag, Band 4, Drogen A-D, 5. Auflage, S. 1084-1102].

Die chemische Synthese von Curcumin ist u.a. beschrieben in Heterocycles 1977, 7, 241-246; Liebig Ann. Chem. 1985, 1557-1569 und Org. Prep. Proced. Int. 1994, 26, 674-677.

H. Tonnesen et al. in Acta Chem. Scand. B 36 (7), 1982, 475-479 beschreiben die Kristallstruktur von aus Ethanol/Wasser umkristallisiertem Curcumin.

WO 91/06292 beschreibt ein Verfahren zur Mahlung von Curcumin in Gegenwart eines Hydrokolloids.

Curcumin-Lösungen oder Gelbwurz-Oleoharze haben jedoch den Nachteil, daß sie an der Luft ziemlich schnell verblassen. Aufgrund dieser mangelnden Stabilität und seiner Wasserunlöslichkeit ist die Verwendung von Curcumin in der Lebensmittelindustrie und in pharmazeutischen Zubereitungen stark eingeschränkt. Des weiteren ist die Bioverfügbarkeit von Curcumin bislang nicht zufriedenstellend.

Es war daher Aufgabe der vorliegenden Erfindung, neue und stabile Curcumin-Zubereitungen bereitzustellen, die die oben genannten Nachteile nicht aufweisen.

Diese Aufgabe wurde nun gelöst durch Curcumin-Formulierungen, enthaltend Curcumin der Form A, dessen Röntgendiffraktogramm eine neuartige Linienintensitätsverteilung aufweist. Die erfindungsgemäßen Formulierungen besitzen als intensivste Reflexionslinie im Röntgen-Diffraktogramm eine Linie im Winkelbereich zwischen 2 Θ = 13° und 2 Θ = 15°, bevorzugt im Winkelbereich zwischen 2 Θ = 13,5°und 2 Θ = 14,5°. Die Formulierungen wurden dabei einer Röntgenbeugungsanalyse mit CuKα-Strahlung unterzogen [Röntgen Diffraktometer (2 Θ)].

Als intensivste Reflexionslinien sind in diesem Zusammenhang die Linien mit der höchsten Zählrate im Maximum gemeint.

Bei den im Röntgen-Diffraktogramm der erfindungsgemäßen Formulierungen auftretenden Reflexionslinien ist das Verhältnis der Intensität der Reflexionslinie bei 2 Θ = 17° zur Reflexionslinie im Winkelbereich zwischen 2 Θ = 13° und 2 Θ = 15° kleiner 1, bevorzugt kleiner 0,5, besonders bevorzugt kleiner 0,05, ganz besonders bevorzugt kleiner 0,01.

Die neuen Curcumin-Formulierungen sind ferner dadurch gekennzeichnet, daß sie Curcumin der Form A mit einem Kristallinitätsgrad im Bereich größer 3%, bevorzugt größer 10%, besonders bevorzugt im Bereich von 20 bis 95%, ganz besonders bevorzugt im Bereich von 30 bis 80% enthalten. Der Kristallisationsgrad läßt sich dabei ebenfalls durch Röntgenbeugungsmessungen bestimmen.

Unter Curcumin-Formulierungen sind im Rahmen der vorliegenden Erfindung u.a. wäßrige Dispersionen bzw. wäßrige Suspensionen, bei denen Curcumin der Form A die dispergierte Phase bildet, daraus hergestellte Trockenpulver aber auch Emulsionen wie z.B. o/w/o-Emulsionen zu verstehen. Bevorzugte Formulierungen sind Trockenpulver und wäßrige Dispersionen.

Der Gehalt an Curcumin der Form A in den erfindungsgemäßen Formulierungen liegt im Bereich von 0,1 bis 50 Gew.-%, bevorzugt im Bereich von 2 bis 30 Gew.-%, besonders bevorzugt im Bereich von 5 bis 20 Gew.-%, ganz besonders bevorzugt im Bereich von 8 bis 15 Gew.-%, bezogen auf die Trockenmasse der Formulierungen.

Die mittlere Teilchengröße von Curcumin der Form A in der wäßrigen Dispersion als auch feinverteilt im Trockenpulver liegt je nach Art der Formulierungsmethode im Bereich kleiner 10 µm, bevorzugt im Bereich kleiner 5 µm, besonders bevorzugt im Bereich von 0,01 bis 2 µm, ganz besonders bevorzugt im Bereich von 0,05 bis 2 µm.

Zur Erhöhung der Stabilität der o.g. Curcumin-Formulierungen ist es vorteilhaft, zusätzlich zum Wirkstoff Schutzkolloide, Weichmacher und/oder Stabilisatoren in die Formulierung einzuarbeiten.

Geeignete Schutzkolloide sind sowohl elektrisch geladene Polymere (Polyelektrolyte) als auch neutrale Polymere. Typische Beispiele sind u.a. Gelatine wie Rinder-, Schweine- oder Fischgelatine, Stärke, Dextrin, Pflanzenproteine wie Sojaproteine, die gegebenenfalls hydrolysiert sein können, Pektin, Guar gum, Xanthan, Gummi-Arabikum, Kasein, Kaseinat oder Mischungen davon. Es können aber auch Polyvinylalkohol, Polyvinylpyrrolidon, Methylcellulose, Carboxymethylcellulose, Hydroxypropylcellulose, Blätterschellack und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen.

Bevorzugte Schutzkolloide sind Verbindungen, ausgewählt aus der Gruppe, bestehend aus Gelatine wie Rinder-, Schweine- und Fischgelatine, Pflanzenproteine, Pektin, Kasein, Kaseinat, Gummi Arabicum und Schellack. Besonders bevorzugt eingesetzte Schutzkolloide sind wäßrige Lösungen von Pektin, Kasein, Kaseinat, Gummi Arabicum und/oder Fischgelatine.

Zur Erhöhung der mechanischen Stabilität des Endproduktes ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin, aber auch Polymere wie Polyvinylalkohol oder Polyvinylpyrrolidon. Bevorzugt verwendete Weichmacher sind Saccharose, Sorbit und Lactose.

Das Verhältnis von Schutzkolloid und Weichmacher zu Curcumin wird im allgemeinen so gewählt, daß ein Endprodukt erhalten wird, das zwischen 0,1 und 50 Gew.-% Curcumin der Form A, 10 bis 50 Gew.-%, bevorzugt 15 bis 35 Gew.-% eines Schutzkolloids, 20 bis 70 Gew.-%, bevorzugt 30 bis 60 Gew.-% eines Weichmachers, wobei sich alle Prozentangaben auf die Trockenmasse der Formulierung beziehen und die Summe der Prozentangaben der Einzelkomponenten 100% ergibt.

Zur Erhöhung der Stabilität des Wirkstoffes gegen oxidativen Abbau kann es vorteilhaft sein, 0 bis 10 Gew.-%, bevorzugt 0,5 bis 7,5 Gew.-%, bezogen auf die Trockenmasse der Formulierung, eines oder mehrerer Stabilisatoren wie α-Tocopherol, t-Butylhydroxy-toluol, t-Butylhydroxyanisol, Ascorbinsäure oder Ethoxyquine zuzusetzen.

Weiterhin können Emulgatoren beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.%, vorzugsweise 5 bis 150 Gew.%, besonders bevorzugt 10 bis 80 Gew.%, bezogen auf eingesetztes Curcumin der Form A verwendet werden.

Unter Umständen kann es auch vorteilhaft sein, zusätzlich ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.%, besonders bevorzugt 20 bis 100 Gew.%, bezogen auf Curcumin der Form A zu verwenden.

Neben Curcumin der Form A können die Formulierungen noch zusätzlich 0,1 bis 25 Gew.-%, bezogen auf die Trockenmasse der Formulierungen, bevorzugt 2 bis 20 Gew.-%, besonders bevorzugt 5 bis 10 Gew.-% Curcumin der Form B enthalten, dessen intensivste Reflexionslinie im Röntgen-Diffraktogramm im Winkelbereich zwischen 2 Θ = 16° und 2 Θ = 18° oder zwischen 2 Θ = 25° und 2 Θ = 26° liegt.

Gegenstand der Erfindung ist auch ein Verfahren zur Herstellung der eingangs beschriebenen Curcumin-Formulierungen, enthaltend Curcumin der Form A, dessen intensivste Reflexionslinie im Röntgen-Diffraktogramm im Winkelbereich zwischen 2 Θ = 13° und 2 Θ = 15° liegt, dadurch gekennzeichnet, daß man
a) eine molekulardisperse Lösung von Curcumin in einem mit Wasser mischbaren, organischen Lösungsmittel oder einer Mischung aus Wasser und einem mit Wasser mischbaren, organischen Lösungsmittel herstellt und
b) diese Lösung mit einer wäßrigen Lösung eines Schutzkolloids versetzt, wobei die hydrophobe Phase des Curcumins als nanodisperse Phase entsteht.

Zur Herstellung von Trockenpulvern wird die im Verfahrensschritt b) erhaltene Dispersion in einem zusätzlichen Schritt c) von dem Lösungsmittel und dem Wasser befreit und, gegebenenfalls in Gegenwart eines Überzugsmaterials, getrocknet.

Zur Durchführung des erfindungsgemäßen Verfahrens sind im Schritt a) vor allem wassermischbare, thermisch stabile, flüchtige, nur Kohlenstoff, Wasserstoff und Sauerstoff enthaltene Lösungsmittel wie Alkohole, Ether, Ester, Ketone und Acetale geeignet. Allgemein verwendet man zweckmäßig solche Lösungsmittel, die mindestens zu 10% wassermischbar sind, einen Siedepunkt unter 200°C aufweisen und/oder weniger als 10 Kohlenstoffe haben. Vorzugsweise werden Ethanol, n-Propanol, Isopropanol, 1,2-Butandiol-1-methylether, 1,2-Propandiol-1-n-propylether oder Aceton verwendet.

Das Verfahren ist ferner dadurch gekennzeichnet, daß man der Curcuminlösung im Schritt a) gegebenenfalls weitere Zusatzstoffe, ausgewählt aus der Gruppe, bestehend aus Emulgatoren, Polymeren, insbesondere Schellack, und eßbaren Ölen zusetzt und/oder daß im Schritt b) die wäßrige Schutzkolloidlösung zusätzlich mindestens einen Weichmacher enthält. Es ist aber auch möglich, einen oder mehrere Emulgatoren der Schutzkolloidlösung zuzusetzen. Zur Beschreibung dieser Zusatzstoffe sei auf die bereits eingangs gemachten Erläuterungen hingewiesen.

In einer bevorzugten Ausführungsform des Verfahrens wird im Schritt a) die molekulardisperse Curcuminlösung bei Temperaturen größer 30°C, bevorzugt bei Temperaturen im Bereich von 50°C bis 240°C, besonders bevorzugt im Bereich von 140°C bis 180°C hergestellt und unmittelbar anschließend im Schritt b) mit der wäßrigen Lösung des Schutzkolloids versetzt, wobei sich eine Mischungstemperatur von etwa 35°C bis 80°C, besonders bevorzugt von 55°C bis 70°C einstellt.

Da die Einwirkung hoher Temperaturen zu unerwünschten Wirkstoffverlusten an Curcumin führen kann, löst man das Curcumin möglichst rasch, beispielsweise im Sekundenbereich, z.B. in 0,1 bis 10 Sekunden, besonders bevorzugt in weniger als 1 Sekunde. Zur raschen Herstellung der molekulardispersen Lösung kann die Anwendung von erhöhtem Druck, z.B. im Bereich von 20 bar bis 100 bar, vorzugsweise 30 bis 80 bar, vorteilhaft sein.

Je nach Art und Menge des verwendeten Schutzkolloids erhält man nach dem Verfahrensschritt b) eine tiefgefärbte wäßrige Dispersion von nanopartikulär dispergiertem Curcumin der Form A.

Zur Herstellung eines Trockenpulvers kann die Entfernung des Lösungsmittels beispielsweise durch Extraktion mit einem mit Wasser nicht mischbaren Lösungsmittel oder, je nach Siedepunkt in an sich bekannter Weise, z.B. durch Destillation, gegebenenfalls unter vermindertem Druck, erfolgen. In diesem Fall hat es sich als zweckmäßig und möglich erwiesen, das bei Verwendung von Isopropanol erhaltene Azeotrop ohne Wasserentfernung unmittelbar als Lösungsmittel einzusetzen. Vorzugsweise erfolgt die Lösungsmittelabtrennung jedoch gemeinsam mit der Entfernung des Wassers durch Sprühtrocknung oder Sprühgranulation.

Die Überführung der wäßrigen Curcumin-Dispersion in ein Trockenpulver kann gegebenenfalls auch in Gegenwart eines Überzugsmaterials erfolgen. Als Überzugsmittel eignen sich u.a. Maisstärke, Kieselsäure oder auch Tricalciumphosphat.

Je nach Trocknungsmethode weisen die Curcumin-haltigen Trockenpulver eine mittlere Partikelgröße von 100 bis 1000 µm bevorzugt 200 bis 800 µm, besonders bevorzugt 250 bis 600 µm.

Hinsichtlich einer genaueren Verfahrens- und Apparatebeschreibung wird an dieser Stelle ausdrücklich auf EP-A-0 065 193 Bezug genommen.

Die wäßrigen Dispersionen, in denen Curcumin der Form A die dispergierte Phase bildet, können analog EP-A-0 845 503 ebenfalls in eine weitere Ölphase einemulgiert werden, so daß sie ein doppeltes Dispersionssystem in Form einer o/w/o-Emulsion bilden. Eine solche Formulierung wiederum hat den Vorteil, daß sie gut mit Ölen mischbar ist und daher für entsprechende Anwendungen in lipophilen Systemen vorteilhaft eingesetzt werden kann.

Die nach dem obigen Verfahren hergestellten Curcumin-Formulierungen zeichnen sich durch eine sehr hohe Farbstabilität und eine verbesserte Bioverfügbarkeit aus. Die erfindungsgemäßen Trockenpulver besitzen darüberhinaus eine im Vergleich zu kristallinem Curcumin sehr gute Redispergierbarkeit, sowohl im warmen als auch im kalten Wasser.

Die erfindungsgemäßen Curcumin-Formulierungen eignen sich u.a. als Zusatzstoff für Lebensmittelzubereitungen und Tierfuttermittel, als Mittel für die Herstellung pharmazeutischer und kosmetischer Zubereitungen sowie für die Herstellung von Nahrungsergänzungspräparaten im Human- und Tierbereich.

Ein typisches Einsatzgebiet im Lebensmittelbereich ist beispielsweise die Verwendung als Färbemittel für Getränke, Milchprodukte wie Käse, Joghurt, Milchmixgetränke oder Milchspeiseeis sowie für Puddingpulver, Senf, Eiprodukte, Salatdressing, Backmischungen, Gewürze und Süßwaren.

Im Futtermittelbereich beispielsweise können die Curcumin-Formulierungen gegebenenfalls zusammen mit Carotinoiden zur Eidotter- und Broilerhautpigmentierung in der Geflügelzucht verwendet werden.

Im Kosmetikbereich können die erfindungsgemäßen Curcumin-Zubereitungen beispielsweise als Farbmittel für dekorative Körperpflegemittel verwendet werden.

Gegenstand der Erfindung sind auch Nahrungsergänzungsmittel, Tierfuttermittel, Lebensmittel sowie pharmazeutische und kosmetische Zubereitungen, enthaltend die oben beschriebenen Curcumin-Formulierungen.

Unter Nahrungsergänzungspräparate sowie pharmazeutische Zubereitungen, die die erfindungsgemäße Curcumin-Formulierung enthalten, sind u.a. Tabletten, Dragees sowie Hart- und Weichgelatinekapseln zu verstehen.

Als Lebensmittel sind beispielsweise Getränke, Milchprodukte wie Käse, Joghurt, Milchmixgetränke oder Milchspeiseeis sowie Puddingpulver, Senf, Eiprodukte, Salatdressing, Backmischungen, Gewürze und Süßwaren gemeint, die die oben beschriebenen Curcumin-Formulierungen enthalten.

Kosmetische Zubereitungen, die die erfindungsgemäßen Curcumin-Formulierungen enthalten können, sind beispielsweise topisch anwendbare Zubereitungen, insbesondere dekorative Körperpflegemittel wie Lippenstifte, Gesichts-Make-up in Form einer Creme, einer Lotion, eines Puders oder auch als Rouge.

Die pharmazeutischen Zubereitungen eignen sich zur Wundbehandlung, als Choleretikum sowie zur Prophylaxe oder Therapie eines zu hohen Cholesterinspiegels.

In den nachfolgenden Beispielen wird die Herstellung der erfindungsgemäßen Curcumin-Formulierungen näher erläutert. Einzelheiten zu der in den Beispielen verwendeten Apparatur finden sich in EP-B-0 065 193.

### Beispiel 1

### Herstellung eines 10 Gew.-%igen Curcumin-Trockenpulvers mit Natrium-Kaseinat und Gelatine als Schutzkolloide

Als Wirkstoffsuspension wurden zunächst 47 g Curcumin in 343 g eines azeotropen Gemischs aus Isopropanol und Wasser eingerührt. Als wäßrige Schutzkolloidlösung wurden 71,7 g Gelatine B 200 Bloom, 36 g Na-Kaseinat, 137 g Saccharose und 4,65 g eines Konservierungssystems, bestehend aus 2,15 g Na-Benzoat, 1,43 g Sorbinsäure, 0,75 g Methylparaben und 0,32 g Propylparaben, in 10820 g Wasser gelöst. Der pH-Wert der Schutzkolloidlösung wurde durch Zugabe von 13 ml einer 1 M NaOH auf pH 6,4 eingestellt.

Die Wirkstoffsuspension wurde auf eine Temperatur von 96°C aufgeheizt und mit einer Flußrate von 2,0 kg/h kontinuierlich mit Isopropanol/Wasser-Azeotrop, das auf eine Temperatur von 217°C gebracht wurde, bei einer Flußrate von 2,7 kg/h vermischt. Dabei löste sich das Curcumin molekular. Diese Curcuminlösung wurde mit der o.g. wäßrigen Schutzkolloidlösung, deren Flußrate 54,8 kg/h betrug, vermischt. Dabei wurde das Curcumin in Form von Kern/ Schale-Teilchen als Hydrosol präzipitiert.

Die Curcumin-Teilchen wiesen eine Teilchengröße von 194 nm bei einer Varianz von 41% auf. Der Curcumingehalt im Hydrosol betrug 0,32% bei einem E1/1-Wert von 83. Das Hydrosol wurde anschließend in einem Dünnfilmverdampfer auf ca. 38% Feststoffgehalt aufkonzentriert und dann über Doppelemulgierung zu einem Trockenpulver aufgearbeitet. Der Curcumingehalt im Trockenpulver betrug dann 10,4 Gew.-%.

### Beispiel 2:

### Herstellung eines 10 Gew.-%igen Curcumin-Trockenpulvers mit einer Gelatinemischung als Schutzkolloide

Wirkstoffsuspension und Schutzkolloidlösung wurden wie in Beispiel 1 angesetzt, jedoch wurden in der Schutzkolloidlösung anstelle von 36 g Na-Caseinat 36 g niedermolekulare Gelatine vom Typ Gelita Sol P gelöst. Der pH-Wert der Schutzkolloidlösung wird durch Zugabe von 18 ml 1 M NaOH auf pH 6,4 eingestellt.

Die Löse- und Präzipitationsbedingungen entsprachen denen von Beispiel 1.

Die Curcumin-Teilchen wiesen eine Teilchengröße von 275 nm bei einer Varianz von 42% auf. Der Curcumingehalt im Hydrosol betrug 0,32% bei einem E1/1-Wert von 64. Das Hydrosol wurde anschließend in einem Dünnfilmverdampfer (Sambay) auf 37% Feststoffgehalt aufkonzentriert und dann über Doppelemulgierung zu einem Trockenpulver aufgearbeitet. Der Curcumingehalt im Trockenpulver betrug dann 10,7%.

### Beispiel 3:

### Herstellung eines 12 Gew.-%igen Curcumin-Trockenpulvers mit Na-Kaseinat als Schutzkolloide

Die Wirkstoffsuspension wurde analog Beispiel 1 angesetzt. Als wäßrige Schutzkolloidlösung wurden 36 g Na-Kaseinat, 137 g Saccharose und 4,65 g des Konservierungssystems aus Beispiel 1 in 10820 g Wasser gelöst. Der pH-Wert der Schutzkolloidlösung wurde durch Zugabe von 10 ml 1 M NaOH auf pH 6,5 eingestellt.

Die Löse- und Präzipitationsbedingungen waren wie folgt: Wirkstoffsuspension 97°C bei einer Flußrate von 2,1 kg/h, Isopropanol/ Wasser-Azeotrop 220°C bei 2,4 kg/h und Schutzkolloidlösung mit 55,3 kg/h. Dabei wurde das Curcumin wie in Beispiel 1 in Form von Kern/Schale-Teilchen als Hydrosol präzipitiert.

Die Curcumin-Teilchen wiesen eine Teilchengröße von 219 nm bei einer Varianz von 46% auf. Der Curcumingehalt im Hydrosol betrug 0,31% bei einem E1/1-Wert von 67. Das Hydrosol wurde anschließend in einem Dünnfilmverdampfer (Sambay) auf 45% Feststoffgehalt aufkonzentriert, mit 19,3 g Gelatine B 200 Bloom aufgelackt und dann über Doppelemulgierung zu einem Trockenpulver aufgearbeitet. Der Curcumingehalt im Trockenpulver betrug 12,3%.

### Beispiel 4:

### Herstellung eines 12 Gew.-%igen Curcumin-Trockenpulvers mit Na-Kaseinat als Schutzkolloid und Ascorbylpalmitat

Die Wirkstoffsuspension wurde wie in Beispiel 1 angesetzt. Als wäßrige Schutzkolloidlösung wurden 39 g Na-Kaseinat und 1,1 g Ascorbylpalmitat in 10820 g Wasser gelöst. Der pH-Wert der Schutzkolloidlösung stellte sich bei pH 6,9 ohne Zugabe von NaOH ein.

Die Löse- und Präzipitationsbedingungen waren wie folgt: Wirkstoffsuspension 95°C bei einer Flußrate von 2,1 kg/h, Isopropanol/ Wasser-Azeotrop 219°C bei 2,5 kg/h und Schutzkolloidlösung mit 55,4 kg/h. Dabei wurde das Curcumin wie in Beispiel 1 in Form von Kern/Schale-Teilchen als Hydrosol präzipitiert. Das Hydrosol wurde anschließend mit 19,7 g Gelatine B 200 Bloom aufgelackt und in einem Rotationsverdampfer auf 27% Feststoffgehalt aufkonzentriert und dann über Doppelemulgierung zu einem Trockenpulver aufgearbeitet. Der Curcumingehalt im Trockenpulver betrug dann 11,8%.

### Beispiel 5:

### Herstellung eines ca. 12 Gew.-%igen Curcumin-Trockenpulvers mit Na-Kaseinat als Schutzkolloid und Ascorbylpalmitat

Als Wirkstoffsuspension wurden zunächst 47 g Curcumin und 141 g Maiskeimöl in 686 g Isopropanol eingerührt. Als wäßrige Schutzkolloidlösung wurden 36 g Na-Kaseinat und 1,1 g Ascorbylpalmitat in 10820 g Wasser gelöst. Der pH-Wert der Schutzkolloidlösung stellte sich bei pH 6,8 ein.

Die Löse- und Präzipitationsbedingungen waren wie folgt: Wirkstoffsuspension 95°C bei einer Flußrate von 2,1 kg/h, Isopropanol/ Wasser-Azeotrop 219°C bei 2,2 kg/h und Schutzkolloidlösung mit 55,2 kg/h. Dabei wurde das Curcumin mit dem Maiskeimöl wie in Beispiel 1 in Form von Kern/Schale-Teilchen als Hydrosol präzipitiert, wobei die Teilchenkerne sowohl das Curcumin als auch das Öl enthalten.

Die Teilchen wiesen eine Größe von 389 nm bei einer Varianz von 72% auf. Der Curcumingehalt im Hydrosol betrug 0,17% bei einem E1/1-Wert von 25. Das Hydrosol wird anschließend in einem Dünnfilmverdampfer (Sambay) auf 48% Feststoffgehalt aufkonzentriert, mit 10 g Gelatine B 200 Bloom aufgelackt und dann über Doppelemulgierung zu einem Trockenpulver aufgearbeitet. Der Curcumingehalt im Trockenpulver betrug dann 11,6%.

### Beispiel 6:

### Fällung von Curcumin und Blätterschellack als Hydrosol, mit Ascorbylpalmitat und Na-Caseinat

Als Wirkstoffsuspension wurden zunächst 47 g Curcumin und 47 g Blätterschellack in 343 g Isopropanol eingerührt. Als wäßrige Schutzkolloidlösung wurden 36 g Na-Caseinat und 1,1 g Ascorbylpalmitat in 10820 g Wasser gelöst. Der pH-Wert stellte sich dabei auf pH 6,9 ein.

Die Wirkstoffsuspension wurde auf eine Temperatur von 95°C aufgeheizt und mit einer Flußrate von 1,1 kg/h kontinuierlich mit Isopropanol/Wasser-Azeotrop, das auf eine Temperatur von 218°C gebracht wurde, bei einer Flußrate von 0,8 kg/h vermischt. Dabei lösten sich Curcumin und Blätterschellack molekular. Diese Lösung wurde mit der o.g. wäßrigen Schutzkolloidlösung, deren Flußrate 54,8 kg/h betrug, vermischt. Dabei wurden Curcumin und Blätterschellack als Matrixteilchen präzipitiert, die durch Na-Kaseinat als Hydrosol stabilisiert wurden. Das Hydrosol wurde anschließend in einem Rotationsverdampfer auf 31% Feststoffgehalt aufkonzentriert, mit 14,6 g Gelatine B 200 Bloom aufgelackt und dann über Doppelemulgierung zu einem Trockenpulver aufgearbeitet. Der Curcumingehalt im Trockenpulver betrug dann 11,5%.

Die erfindungsgemäß hergestellten Trockenpulver wurden einer Röntgenbeugungsanalyse mit CuKα-Strahlung unterzogen. Die Röntgenweitwinkeldiagramme wurden im Winkelbereich 1°≤ 2Θ ≤ 50° registriert.

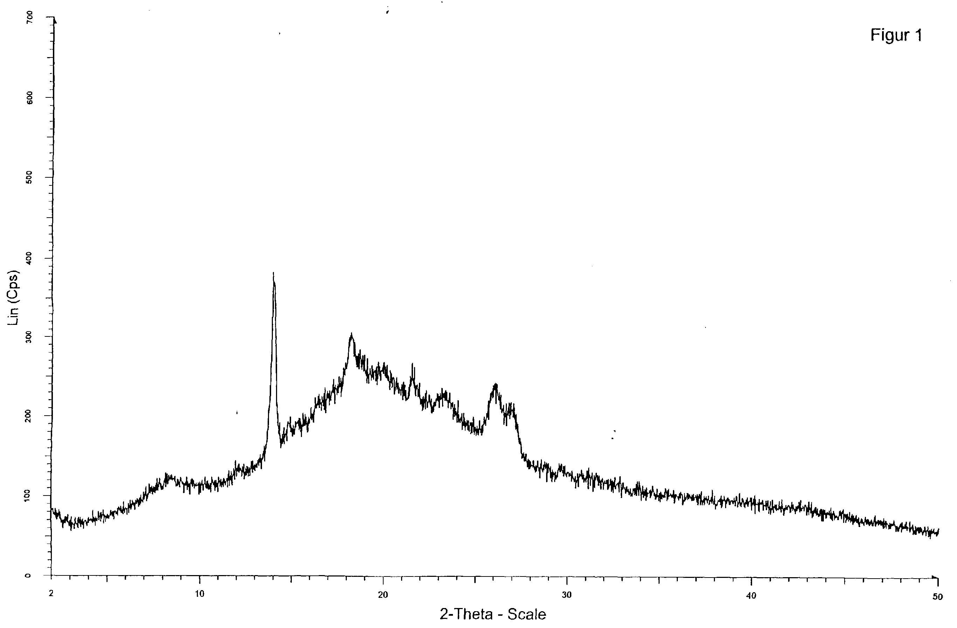

Figur 1 zeigt ein Pulverdiffraktogramm eines erfindungsgemäßen Curcumin-haltigen Trockenpulvers, hergestellt nach Beispiel 4.

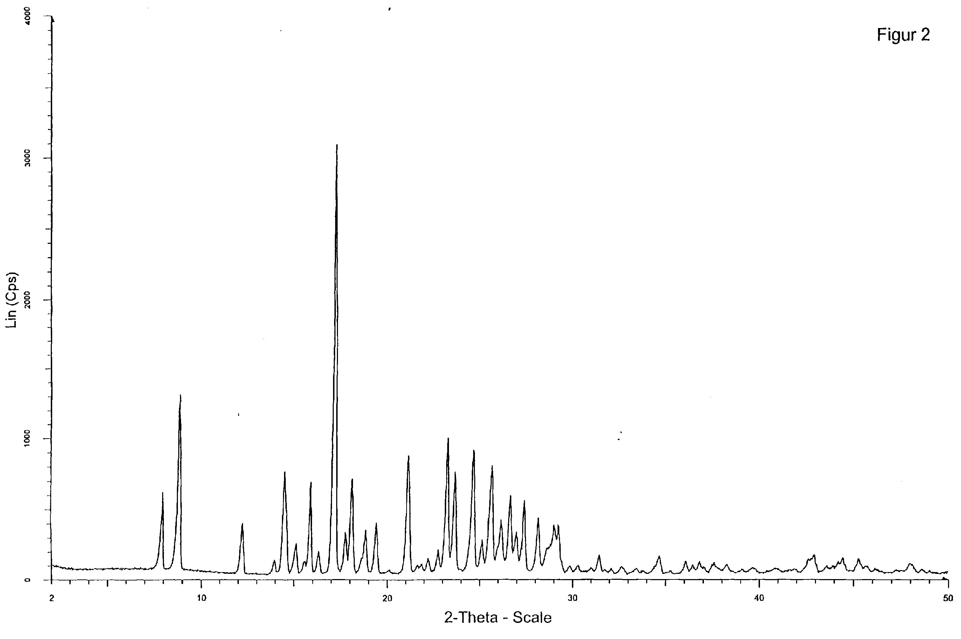

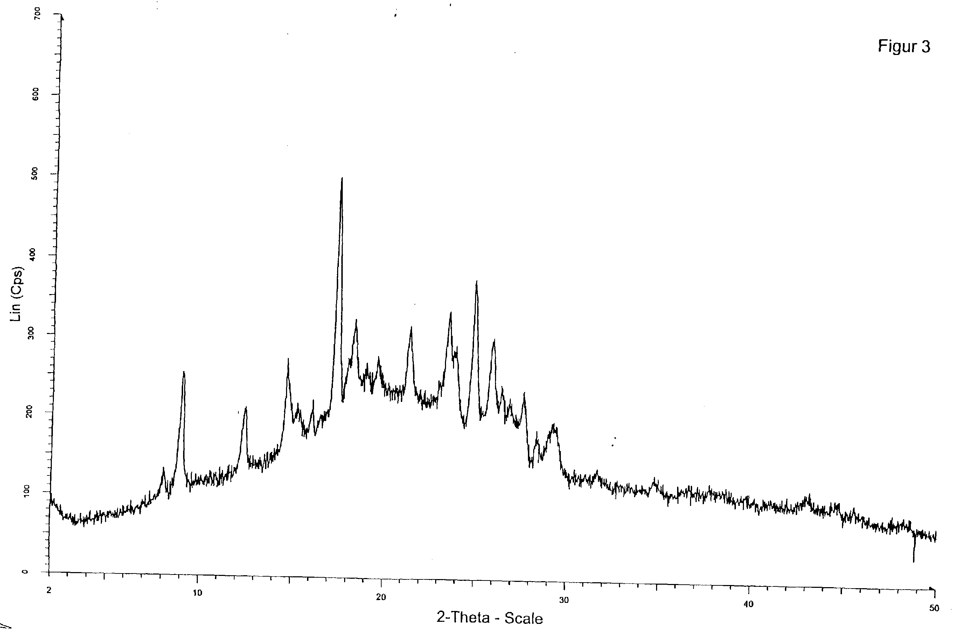

Figur 2 zeigt ein Pulverdiffraktogramm von kristallinem Curcumin i gemäß dem Stand der Technik.

Figur 3 zeigt ein Pulverdiffraktogramm eines Curcumin-haltigen Trockenpulvers, hergestellt nach WO 91/06292 (Beispiel 13).

## Patentansprüche

1. Curcumin-Formulierungen, enthaltend Curcumin der Form A, dessen intensivste Reflexionslinie im Röntgen-Diffraktogramm im Winkelbereich zwischen 2 Θ = 13° und 2 Θ = 15° liegt.

2. Curcumin-Formulierungen nach Anspruch 1, wobei das Verhältnis der Intensität der Reflexionslinie bei 2 Θ = 17° zu der Reflexionslinie im Winkelbereich zwischen 2 Θ = 13° und 2 Θ = 15° kleiner 1 ist.

3. Curcumin-Formulierungen nach einem der Ansprüche 1 oder 2, dadurch gekennzeichnet, daß es sich um wäßrige Dispersionen oder um Trockenpulver handelt.

4. Curcumin-Formulierungen nach einem der Ansprüche 1 bis 3, enthaltend Curcumin der Form A mit einem Kristallinitätsgrad von größer 3%.

5. Curcumin-Formulierungen nach einem der Ansprüche 1 bis 4, enthaltend Curcumin der Form A mit einer Teilchengröße von weniger als 10 µm.

6. Curcumin-Formulierungen nach einem der Ansprüche 1 bis 5, enthaltend 0,1 bis 50 Gew.-% Curcumin der Form A, 10 bis 50 Gew.-% eines Schutzkolloids, 20 bis 70 Gew.-% eines Weichmachers, 0 bis 10 Gew.-% eines Stabilisators, wobei sich alle Prozentangaben auf die Trockenmasse der Formulierung bezieher und die Summe der Prozentangaben der Einzelkomponenten 100 % ergibt.

7. Curcumin-Formulierungen nach Anspruch 6, enthaltend zusätzlich 0,1 bis 25 Gew.-% Curcumin der Form B, dessen intensivste Reflexionslinie im Röntgen-Diffraktogramm im Winkelbereich zwischen 2 Θ = 16° und 2 Θ = 18° oder zwischen 2 Θ = 25° und 2 Θ = 26° liegt.

8. Verfahren zur Herstellung von Curcumin-Formulierungen, definiert gemäß Anspruch 1, dadurch gekennzeichnet, daß man
a) eine molekulardisperse Lösung von Curcumin in einem mit Wasser mischbaren, organischen Lösungsmittel oder einer Mischung aus Wasser und einem mit Wasser mischbaren, organischen Lösungsmittel herstellt und
b) diese Lösung mit einer wäßrigen Lösung eines Schutzkolloids versetzt, wobei die hydrophobe Phase des Curcumins als nanodisperse Phase entsteht.

9. Verfahren nach Anspruch 8, dadurch gekennzeichnet, daß man die im Verfahrensschritt b) erhaltene Dispersion für die Herstellung eines Trockenpulvers in einem zusätzlichen Schritt c) von dem Lösungsmittel und dem Wasser befreit und, gegebenenfalls in Gegenwart eines Überzugsmaterials, trocknet.

10. Verfahren nach einem der Ansprüche 8 oder 9, dadurch gekennzeichnet, daß man der Curcuminlösung im Schritt a) weitere Zusatzstoffe, ausgewählt aus der Gruppe, bestehend aus Emulgatoren, Polymeren, und eßbaren Ölen zusetzt.

11. Verfahren nach einem der Ansprüche 8 bis 10, dadurch gekennzeichnet, daß im Schritt b) die wäßrige Schutzkolloidlösung zusätzlich mindestens einen Weichmacher enthält.

12. Verfahren nach einem der Ansprüche 8 bis 11, dadurch gekennzeichnet, daß man im Schritt a) die Curcuminlösung bei Temperaturen größer 30°C herstellt und unmittelbar anschließend im Schritt b) mit der wäßrigen Lösung des Schutzkolloids versetzt, wobei sich eine Mischungstemperatur von 35°C bis 80°C einstellt.

13. Verwendung der Curcumin-Formulierungen, definiert gemäß einem der Ansprüche 1 bis 7 zur Herstellung von Nahrungsergänzungsmitteln, pharmazeutischen und kosmetischen Zubereitungen sowie als Zusatz zu Lebensmitteln und Tierfuttermitteln.

14. Verwendung nach Anspruch 13 als Färbemittel.

15. Verwendung nach Anspruch 13 zur Herstellung von Nutraceuticals und pharmazeutischen Zubereitungen.

16. Verwendung nach Anspruch 15 zur Vorbeugung oder Behandlung eines zu hohen Cholesterinspiegels.

17. Nahrungsergänzungsmittel, Tierfuttermittel, Lebensmittel sowie pharmazeutische und kosmetische Zubereitungen, enthaltend Curcumin-Formulierungen, definiert gemäß einem der Ansprüche 1 bis 7.
